Europäisches Patentamt

European Patent Office

Office européen des brevets

(19)

(11) Publication number: **0 115 539**
**B1**

(12) # EUROPEAN PATENT SPECIFICATION

(45) Date of publication of patent specification: **29.10.86**

(21) Application number: **83902295.1**

(22) Date of filing: **26.07.83**

(86) International application number:
**PCT/JP83/00241**

(87) International publication number:
**WO 84/00543 16.02.84 Gazette 84/05**

(51) Int. Cl.⁴: **C 07 C 149/247,**
**C 07 C 148/04**

(54) **SINGLE CRYSTALLINE DL-CYSTEINE AND PROCESS FOR THEIR PREPARATION.**

(30) Priority: **29.07.82 JP 131095/82**

(43) Date of publication of application:
**15.08.84 Bulletin 84/33**

(45) Publication of the grant of the patent:
**29.10.86 Bulletin 86/44**

(84) Designated Contracting States:
**FR**

(56) References cited:

**No citations**

(73) Proprietor: **MITSUI TOATSU CHEMICALS,
INCORPORATED**
**2-5 Kasumigaseki 3-chome**
**Chiyoda-Ku Tokyo 100 (JP)**

(72) Inventor: **MITA, Ryuichi**
**529, Shimosakunobe, Takatsu-ku**
**Kawasaki-shi, Kanagawa 213 (JP)**
Inventor: **KATOH, Toshio**
**600-1, Kamisakunobe, Takatsu-ku**
**Kawasaki-shi, Kanagawa 213 (JP)**
Inventor: **HIGUCHI, Chojiro**
**4-5-13, Dai Kamakura-shi**
**Kanagawa 247 (JP)**
Inventor: **YAMAGUCHI, Akihiro**
**1-13-24, Zaimokura, Kamakura-shi**
**Kanagawa 248 (JP)**
Inventor: **OHOKA, Masaharu**
**4-5-45, Kamakura-shi**
**Kanagawa 247 (JP)**

(74) Representative: **Lecca, Jean et al**
**CABINET PLASSERAUD 84, rue d'Amsterdam**
**F-75009 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**0 115 539**

**Description**

Technical field

This invention relates to single-crystal DL-cysteine and a process for producing the same. More particularly, it relates to DL-cysteine having a crystalline nature characterized by the following X-ray diffraction spectrum and a process for producing the same.

| Lattice spacing (Å) | Relative intensity* (I) |
| --- | --- |
| 11.90 | f |
| 5.97 | f |
| 3.97 | f |
| 2.98 | ff |

*) The relative intensity (I) is expressed on the basis of the following, arbitrarily defined criteria: ff=Very intense; f=Intense.

Background art

DL-cysteine is a sulfur-containing amino acid and has wide applications in the preparation of permanent-wave solutions, the manufacture of drugs, and the like.

It is generally known that cysteine is readily oxidized to cystine by atmospheric oxygen or the like. In particular, free DL-cysteine that has been known in the prior art is an amorphous substance having no crystalline form. For this reason, it has the disadvantage of being subject to oxidation during storage and lacking in storage stability. Thus, DL-cysteine is being used for various purposes exclusively in the form of DL-cysteine hydrochloride monohydrate. For example, in the preparation of a cold-wave solution comprising DL-cysteine, DL-cysteine hydrochloride is neutralized with a suitable base and, further, the solution is adjusted to a desired pH value which is generally not less than pH 9. Accordingly, if such a solution could be prepared from free DL-cysteine, only the amount of base used for pH adjustment is needed to economic advantages.

In the prior art, a common method for producing DL-cysteine comprised dissolving the hydrochloric acid or p-toluenesulfonic acid salt of DL-cysteine in methanol and neutralizing the solution with a base such as triethylamine or the like. However, the free DL-cysteine produced by this method is not crystalline but amorphous. This fact raises difficult problems because such free DL-cysteine is readily oxidized to cystine during storage, and therefore, lacks in storage stability.

Disclosure of the invention

It is an object of the present invention to provide single-crystal DL-cysteine having a crystalline nature and hence good storage stability and a process for producing the same.

According to the present invention, there is provided DL-cysteine having the nature of single crystals as is characterized by the above-described X-ray diffraction spectrum. Moreover, there is also provided a process for producing single-crystal DL-cysteine in which a hydrochloric acid or aromatic sulfonic acid salt of DL-cysteine is suspended or dissolved in a solvent mixture comprising water and an organic solvent selected from the group consisting of lower alcohols and cellosolves, and then neutralized with a base to form single-crystal DL-cysteine.

The single-crystal DL-cysteine produced by the process of the present invention is free DL-cysteine having a flaky or short needle-like crystal form and exhibiting good storage stability.

Best mode for carrying out the invention

The single-crystal DL-cysteine of the present invention is DL-cysteine having a crystalline nature characterized by the following X-ray diffraction spectrum:

| Lattice spacing (Å) | Relative intensity* (I) |
| --- | --- |
| 11.90 | f |
| 5.97 | f |
| 3.97 | f |
| 2.98 | ff |

*) The relative intensity (I) is expressed on the basis of the following, arbitrarily defined criteria: ff=Very intense; f=Intense.

According to the process of the present invention, single-crystal DL-cysteine is produced by suspending or dissolving an aromatic sulfonic acid or hydrochloric acid salt of DL-cysteine in a solvent mixture comprising water and an organic solvent selected from the group consisting of lower alcohols and cellosolves, and then neutralizing it with a base.

2

# 0 115 539

As the aromatic sulfonic acid salt of DL-cysteine used in the process of the present invention, its substituted or unsubstituted benzenesulfonic acid salts are generally useful. Specific examples thereof include benzenesulfonic acid, p-toluenesulfonic acid, p-ethylbenzenesulfonic acid, p-chlorobenzenesulfonic acid, m-xylenesulfonic acid, sulfosalicylic acid, m-nitrobenzenesulfonic acid, α-naphtholsulfonic acid and β-naphtholsulfonic acid salts of DL-cysteine. Where the hydrochloric acid salt of DL-cysteine is used, the salt may be in the form of anhydride or monohydrate.

No particular limitation is placed on the type of base used in the process of the present invention. More specifically, a variety of bases are usable provided that, when used to neutralize an aromatic sulfonic acid or hydrochloric acid salt of DL-cysteine, they producing a salt soluble in the water-containing lower alcohol or like solvent used in the process of the present invention. In contrast, bases producing a salt scarcely soluble in the water-containing lower alcohol or like solvent are undesirable because it is difficult to separate the precipitated free DL-cysteine from the salt. Specific examples of the bases useful in the process of the present invention include organic bases such as ethylamine, diethylamine, triethylamine, monoethanolamine, triethanolamine, pyridine, piperidine, etc.; alkali metal or alklaine earth metal hydroxides such as lithium hydroxide, sodium hydroxide, potassium hydroxide, calcium hydroxide, barium hydroxide, etc.; alkali metal or alkaline earth metal carbonates such as lithium carbonate, sodium carbonate, potassium carbonate, calcium carbonate, barium carbonate, etc.; and ammonia.

The process of the present invention is carried out in a solvent mixture comprising water and an organic solvent selected from the group consisting of lower alcohols and cellosolves. Organic solvents having miscibility with water are useful in the process of the present invention. Specific examples thereof include lower alcohols having 1 to 4 carbon atoms, such as methanol, ethanol, n-propanol, isopropanol, tert-butanol, etc.; and cellosolves such as methyl cellosolve, ethyl cellosolve, etc.

In the process of the present invention these organic solvents must contain water. The amount of water contained therein should be not less than 5% by weight. Although its upper limit is not clearly defined, unduly large amounts of water may reduce the recovery of DL-cysteine. Thus, the water is usually used in an amount of 5 to 60% by weight, the preferred range being 10 to 50% by weight. If the amount of water used is less than 5% by weight, the resulting free DL-cysteine will be an amorphous substance having no crystalline form and lacking in storage stability.

No particular limitation is placed on the amount of solvent mixture used. However, in view of the actual practice of the reaction and the recovery of DL-cysteine, the solvent mixture is generally used in an amount equaling 0.5 to 10 times, preferably 1 to 6 times, the weight of the aromatic sulfonic acid salt of DL-cysteine or in an amount equaling 1 to 20 times, preferably 2 to 10 times, the weight of the hydrochloric acid salt of DL-cysteine.

In carrying out the process of the present invention, an aromatic sulfonic acid or hydrochloric acid salt of DL-cysteine is dissolved or suspended in a solvent mixture as defined above, Then, a base is added thereto, drop by drop or all at once, or blown therethrough at a temperature of −20 to 80°C, preferably 0 to 60°C, to neutralize the solution or suspension and thereby precipitate free DL-cysteine. After completion of the neutralization, the solution or suspension is cooled as desired and the precipitated free DL-cysteine is separated by filtration. In order to prevent the oxidation of the cysteine to cystine, the neutralization is preferably carried out under an atmosphere of an inert gas such as nitrogen, argon or the like. In this manner, there is obtained free DL-cysteine having a flaky or short needle-like crystal form and exhibiting good storage stability.

The present invention is further illustrated by the following examples.

Example 1

29.3 g of DL-cysteine p-toluenesulfonate was suspended in 70 ml of methanol containing 10% by weight of water. Under an atmosphere of nitrogen, the suspension was neutralized by adding, drop by drop, 10.1 g of triethylamine at 0—5°C with stirring. After stirring at the same temperature for 1 hour, the precipitated crystals were separated by filtration, washed with methanol and then dried in vacuo to obtain free DL-cysteine in the form of colorless flakes. Its yield was 11.0 g (90.9% recovery).

When measured with a Cu:Ni X-ray tube (20 kV, 10 mA) and at λ=1.5418, the X-ray diffraction spectrum of the crystals showed the following X-ray diffraction pattern.

3

**0 115 539**

| Lattice spacing (Å) | Relative intensity (I) |
|---|---|
| 11.90 | 0.57 |
| 5.97 | 0.34 |
| 4.55 | 0.01 |
| 4.20 | 0.03 |
| 3.97 | 0.44 |
| 3.82 | 0.03 |
| 3.79 | 0.04 |
| 3.71 | 0.03 |
| 3.41 | 0.01 |
| 3.18 | 0.04 |
| 3.08 | 0.03 |
| 2.98 | 1.00 |
| 2.60 | 0.02 |
| 2.52 | 0.03 |
| 2.39 | 0.03 |
| 2.25 | 0.05 |

Comparative example

29.3 g of DL-cysteine p-toluenesulfonate was suspended in 80 ml of methanol. Under an atmosphere of nitrogen, the suspension was neutralized by adding, drop by drop, 10.1 g of triethylamine at 0—5°C with stirring. After stirring at the same temperature for 1 hour, the resulting precipitate was separated by filtration, washed with methanol and then dried in vacuo to obtain free DL-cysteine in the form of colorless powder. Its yield was 11.6 g (96% recovery).

When measured in the same manner as in Example 1, the X-ray diffraction spectrum of the DL-cysteine power showed the following pattern.

| Lattice spacing (Å) | Relative intensity (I) |
|---|---|
| 11.90 | 0.37 |
| 5.97 | 0.21 |
| 4.93 | 0.08 |
| 4.55 | 0.28 |
| 4.20 | 1.00 |
| 3.97 | 0.21 |
| 3.82 | 0.29 |
| 3.79 | 0.49 |
| 3.75 | 0.37 |
| 3.71 | 0.26 |
| 3.41 | 0.51 |
| 3.18 | 0.24 |
| 3.08 | 0.11 |
| 2.98 | 0.61 |
| 2.68 | 0.16 |
| 2.60 | 0.08 |
| 2.52 | 0.05 |
| 2.39 | 0.02 |
| 2.36 | 0.09 |
| 2.29 | 0.12 |
| 2.25 | 0.16 |

Example 2

29.3 g of DL-cysteine p-toluenesulfonate was suspended in 88 ml of isopropanol containing 40% by weight of water. Under an atmosphere of nitrogen, the suspension was neutralized by adding, drop by drop, 10.1 g of triethylamine at 20—25°C with stirring. After stirring at the same temperature for 1 hour, the precipitated crystals were separated by filtration, washed with isopropanol and then dried in vacuo to obtain free DL-cysteine in the form of colorless flaky crystals. Its yield was 10.9 g (90.1% recovery).

The X-ray diffraction pattern of the crystals was substantially the same as that observed in Example 1.

Example 3

The procedure of Example 2 was repeated except that 31.3 g of DL-cysteine p-chlorobenzenesulfonate was used in place of the DL-cysteine p-toluenesulfonate and 88 ml of isopropanol containing 30% by weight of water was used as the solvent. Thus, there was obtained 11.4 g of free DL-cysteine in the form of colorless flaky crystals.

4

Again, the X-ray diffraction pattern of the crystals was substantially the same as that observed in Example 1.

Example 4

52.7 g of DL-cysteine hydrochloride monohydrate was dissolved in 240 ml of methanol containing 10% by weight of water. Under an atmoshere of nitrogen, the solution was neutralized by adding, drop by drop, 30.3 g of triethylamine at 20—25°C with stirring over a period of about 20 minutes. After stirring at the same temperature for 1 hour, the precipitated crystals were separated by filtration, washed with methanol and then dried in vacuo to obtain 35.1 g of free DL-cysteine in the form of colorless, short needle-like crystals. Upon analysis by highspeed liquid chromatography, the product was found to have a purity of 100% and contain no cystine.

When measured with a Cu:Ni X-ray tube (25 kV, 14 mA) and at $\lambda = 1.5418$, the X-ray diffraction spectrum of the DL-cysteine crystals showed the following X-ray diffraction pattern.

| Lattice spacing (Å) | Relative intensity (I) |
|---|---|
| 11.90 | 0.63 |
| 5.97 | 0.33 |
| 4.55 | 0.03 |
| 4.20 | 0.07 |
| 3.97 | 0.39 |
| 3.82 | 0.06 |
| 3.79 | 0.12 |
| 3.75 | 0.04 |
| 3.71 | 0.05 |
| 3.41 | 0.03 |
| 3.18 | 0.05 |
| 3.08 | 0.05 |
| 2.98 | 1.00 |
| 2.68 | 0.01 |
| 2.60 | 0.01 |
| 2.52 | 0.03 |

Example 5

The procedure of Example 4 was repeated, except that the water-containing methanol was replaced by 240 ml of isopropanol containing 40% by weight of water and the triethylamine was replaced by 23.7 g of pyridine. Thus, there was obtained 32.5 g of free DL-cysteine in the form of colorless flaky crystals. Its purity was 100%, and the X-ray diffraction pattern of the crystals was substantially the same as that observed in Example 4.

Example 6

The procedure of Example 4 was repeated, except that the triethylamine was replaced by 18.5 g of 28% aqueous ammonia. Thus, there was obtained 33.8 g of free DL-cysteine in the form of short needle-like crystals. Its purity was 100%, and the X-ray diffraction pattern of these crystals was substantially the same as that observed in Example 4.

Example 7

52.7 g of DL-cysteine hydrochloride monohydrate was suspended in 200 ml of methanol containing 20% by weight of water. Under an atmosphere of nitrogen, the solution was neutralized by adding 12.6 g of lithium hydroxide, little by little, at 20—25°C with stirring. After stirring at the same temperature for 1 hour, the precipitated crystals were separated by filtration, washed with methanol and then dried in vacuo to obtain free DL-cysteine in the form of colorless flaky crystals. Its yield was 32.7 g (90% recovery) and its purity was 100%. The X-ray diffraction pattern of the crystals was substantially the same as that observed in Example 4.

Reference example

The free DL-cysteine obtained in Example 1 and that obtained in Comparative Example were tested for storage stability by placing them in crystallizing dishes made of glass and allowing them to stand under ordinary room conditions. The results thus obtained are shown in Table 1.

**0 115 539**

TABLE 1

| Sample | Formation of Cystine after 30 days (wt.%)* |
|---|---|
| Example 1 | 0 |
| Comparative example | 5 |

*) Analyzed by high-speed liquid chromatography.

**Claims**

1. Single-crystal DL-cysteine characterized by an X-ray diffraction spectrum in which intense diffracted rays appear at the following lattice spacings:

| Lattice spacing (A) | Relative intensity (I) |
|---|---|
| 11.90 | f |
| 5.97 | f |
| 3.97 | f |
| 2.98 | ff |

where, in the column of the relative intensity (I), f means that the spectral intensity is highly and ff means that the spectral intensity is very high.

2. A process for producing single-crystal DL-cysteine which comprises suspending or dissolving an aromatic sulfonic acid or hydrochloric acid salt of DL-cysteine in a solvent mixture comprising water and an organic solvent selected from the group consisting of alcohols having 1 to 4 carbon atoms, methyl cellosolve and ethyl cellosolve, and then neutralizing with a base.

3. A process as claimed in claim 2, wherein the content of water in the solvent mixture ranges from 5 to 60% by weight.

4. A process as claimed in claim 2 or 3, wherein the base is at least one compound selected from the group consisting of ethylamine, diethylamine, triethylamine, monoethanolamine, triethanolamine, pyridine and piperidine.

5. A process as claimed in claim 2 or 3, wherein the base is at least one compound selected from the group consisting of lithium hydroxide, sodium hydroxide, potassium hydroxide, calcium hydroxide, barium hydroxide, lithium carbonate, sodium carbonate, potassium carbonate, calcium carbonate, barium carbonate and ammonia.

6. A process as claimed in any of claims 2 to 5, wherein the neutralization is carried out at a temperature of −20 to 80°C.

**Patentansprüche**

1. Einzelkristall-DL-Cystein, gekennzeichnet durch ein Röntgenstrahlen-Beugungsspektrum, in dem intensive Beugungsstrahlen bei den folgenden Gitterabständen auftreten:

| Gitterstand (A) | Relative intensität (I) |
|---|---|
| 11,90 | f |
| 5,97 | f |
| 3,97 | f |
| 2,98 | ff |

wobei in der Spalte der relative Intensität (I) f eine hohe spektrale Intensität und ff eine sehr hohe spektrale Intensität bedeutet.

2. Verfahren zur Herstellung von Einzelkristall-DL-Cystein, dadurch gekennzeichnet, daß man ein aromatisches Sulfonsäure- oder Salzsäuresalz von DL-Cystein in einem Lösungsmittelgemisch aus Wasser und einem organischen Lösungsmittel aus der Gruppe Alkohole mit 1 bis 4 Kohlenstoffatomen, Äthylenglykolmonomethyläther und Äthylenglykolmonoäther suspendiert oder löst und dann mit einer Base neutralisiert.

3. Verfahren nach Anspruch 2, in dem der Wassergehalt des Lösungsmittelgemisches im Bereich von 5 bis 60 Gew.-% liegt.

4. Verfahren nach Anspruch 2 oder 3, in dem als Base mindestens eine Verbindung aus der Gruppe Äthylamin, Di-äthylamin, Triäthylamin, Monoäthanolamin, Triäthanolamin, Pyridin und Piperidin eingesetzt wird.

6

5. Verfahren nach Anspruch 2 oder 3, in dem als Base mindestens eine Verbindung aus der Gruppe Lithiumhydroxid, Natriumhydroxid, Kaliumhydroxid, Calciumhydroxid, Bariumhydroxid, Lithiumcarbonat, Natriumcarbonat, Kaliumcarbonat, Calciumcarbonat, Bariumcarbonat und Ammoniak eingesetzt wird.

6. Verfahren nach einem der Ansprüche 2 bis 5, in dem die Neutralisation bei einer Temperatur von −20 bis 80°C ausgeführt wird.

**Revendications**

1. DL-cystéine monocristalline caractérisée par un spectre de diffraction des rayons X dans lequel des rayons à diffraction intense apparaissent aux distances réticulaires suivantes:

| Distance réticulaire (Å) | Intensité relative* (I) |
|---|---|
| 11,90 | f |
| 5,97 | f |
| 3,97 | f |
| 2,98 | ff |

où, dans la colonne de l'intensité relative (I), f indique que l'intensité spectrale est élevée et ff indique que l'intensité spectrale est très élevée.

2. Un procédé pour la production de DL-cystéine monocristalline qui comprend la mise en suspension ou la dissolution d'un sel d'acide sulfonique aromatique ou d'un sel d'acide chlorhydrique de la DL-cystéine dans un mélange solvant comprenant de l'eau et un solvant organique choisi dans le groupe constitué par les alcools ayant 1 à 4 atomes de carbone, le méthylcellosolve et l'éthylcellosolve puis la neutralisation avec une base.

3. Un procédé comme revendiqué dans la revendication 2 dans lequel la teneur en eau du mélange solvant est comprise dans la gamme de 5 à 60% en poids.

4. Un procédé comme revendiqué dans la revendication 2 ou 3 dans lequel la base est au moins un composé choisi dans le groupe constitué par l'éthylamine, la diéthylamine, la triéthylamine, la monoéthanolamine, la triéthanolamine, la pyridine et la pipéridine.

5. Un procédé comme revendiqué dans la revendication 2 ou 3 dans lequel la base est au moins un composé choisi dans le groupe constitué par l'hydroxyde de lithium, l'hydroxyde de sodium, l'hydroxyde de potassium, l'hydroxyde de calcium, l'hydroxyde de baryum, le carbonate de lithium, le carbonate de sodium, le carbonate de potassium, le carbonate de calcium, le carbonate de baryum et l'ammoniac.

6. Un procédé comme revendiqué dans l'une quelconque des revendications 2 à 5 dans lequel la neutralisation est effectuée à une température de −20 à 80°C.